# EUROPEAN PATENT SPECIFICATION

(11) **EP 3 822 353 B1**
(45) Date of publication and mention of the grant of the patent: **27.08.2025**
(21) Application number: 19382997.5
(22) Date of filing: 13.11.2019
(51) Int. Cl.: C12N 15/67

(54) **METHOD FOR THE PRODUCTION OF DSRNA**
VERFAHREN ZUR HERSTELLUNG VON DSRNA
PROCÉDÉ DE PRODUCTION D'ARNDB

(43) Date of publication of application: 19.05.2021
(73) Proprietor: Consejo Superior de Investigaciones Científicas (CSIC), 28006 Madrid (ES); Universitat Politècnica de València, 46022 Valencia (ES)
(72) Inventor: DARÓS ARNAU, José Antonio, 46022 Valencia (ES); ORTOLÁ NAVARRO, Beltrán, 46022 Valencia (ES); CORDERO CUCART, María Teresa, 46022 Valencia (ES)
(74) Representative: Pons IP

(56) References cited:
- JOSÉ-ANTONIO DARÒS ET AL: "A viroid-derived system to produce large amounts of recombinant RNA in Escherichia coli", SCIENTIFIC REPORTS, vol. 8, no. 1, 30 January 2018 (2018-01-30), XP055685866, DOI: 10.1038/s41598-018-20314-3
- TERESA CORDERO ET AL: "Mutational Analysis of Eggplant Latent Viroid RNA Circularization by the Eggplant tRNA Ligase in Escherichia coli", FRONTIERS IN MICROBIOLOGY, vol. 9, 5 April 2018 (2018-04-05), XP055685893, ISSN: 1664-302X, DOI: 10.3389/fmicb.2018.00635
- FENG GUO ET AL: "In vivo selection of better self-splicing introns in Escherichia coli: The role of the P1 extension helix of the Tetrahymena intron", RNA, vol. 8, no. 5, 1 May 2002 (2002-05-01), US, pages 647 - 658, XP055685980, ISSN: 1355-8382, DOI: 10.1017/S1355838202029011
- J. ROMAN ET AL: "Integration of the Tetrahymena group I intron into bacterial rRNA by reverse splicing in vivo", PROCEEDINGS OF THE NATIONAL ACADEMY OF SCIENCES, vol. 95, no. 5, 3 March 1998 (1998-03-03), US, pages 2134 - 2139, XP055685989, ISSN: 0027-8424, DOI: 10.1073/pnas.95.5.2134
- G. DINTER-GOTTLIEB: "Viroids and virusoids are related to group I introns.", PROCEEDINGS OF THE NATIONAL ACADEMY OF SCIENCES, vol. 83, no. 17, 1 September 1986 (1986-09-01), US, pages 6250 - 6254, XP055685971, ISSN: 0027-8424, DOI: 10.1073/pnas.83.17.6250
- ORTOLÁ BELTRÁN ET AL: "Intron-assisted, viroid-based production of insecticidal circular double-stranded RNA in Escherichia coli", RNA BIOLOGY, 20 January 2021 (2021-01-20), pages 1 - 12, XP055778567, ISSN: 1547-6286, Retrieved from the Internet <URL:https://www.tandfonline.com/doi/pdf/10.1080/15476286.2021.1872962> DOI: 10.1080/15476286.2021.1872962

## Description

The present invention relates to a nucleotide sequence comprising a) the cDNAs of two strands of a target double-stranded RNA (dsRNA) separated by a type I autocatalytic intron flanked by exons, and b) a plant Avsunviroidae sequence, wherein element (a) is inserted in the plant Avsunviroidae sequence. The expression of this nucleotide sequence in a host cell, such as *Escherichia coli,* along with a tRNA ligase, allows the production of large amounts of dsRNA specific for a target gene. This dsRNA can be used in the interfering RNA technology for silencing gene expression. Thus, the present invention belongs to the field of biotechnology, specifically, to the field of the production of recombinant RNA.

### BACKGROUND ART

Gene silencing mediated by RNA or RNA interference (RNAi) is a natural defense mechanism against genetic elements, both exogenous (for example, viruses) and endogenous (for example, transposons), which in most eukaryotic organisms has evolved to become an important regulatory way of gene expression. RNAi is triggered by the presence of double-stranded RNA (dsRNA) and has the ultimate consequence of silencing or suppressing homologous messenger RNA (mRNA). Very briefly, the dsRNA is processed by the Dicer endonucleases to produce small RNA (sRNA) that is recognized by the RNase H Argonaute (Ago), which carries one of the strands of the sRNA to form the RNA-induced silencing complex (RISC). In the cell, this complex traces its target RNA based on the complementarity of the sRNA and, when it finds it, it cuts it down inactivating it or it joins it, inhibiting its translation. In some taxonomic groups such as nematodes or plants, an RNA-dependent RNA polymerase (RDR) activity uses sRNA as a primer to produce more homologous dsRNA, which feeds the cycle from its inception and amplifies the interfering signal.

RNAi can be efficiently induced by the administration of exogenous RNA, so it has been widely used in basic research to study gene function in many classes of organisms. At the same time, its conceptual simplicity (highly specific gene suppression based on simple base complementarity) has driven the development of a multitude of promising biotechnological applications. One of them is the use of RNA against pests: the ingestion of dsRNAs by nematodes, insects and other arthropods induces the silencing of endogenous homologous genes, causing their death or affecting their development, mobility or feeding behavior, reducing in any case the damage they cause. The silencing induced by dsRNAs has also been demonstrated in pathogens such as viruses and fungi. In this way, RNA, a natural compound that is harmless to the environment, has become a promising agent against pests and pathogens. In addition, its mechanism of action based on base complementarity makes it highly specific and easily adaptable to any new species or lineage of pest or emerging pathogen. Its use in agricultural crops has aroused great interest but its potential goes beyond agricultural biotechnology.

The biotechnological use of dsRNAs requires, however, systems capable of producing large quantities of this type of compound quickly and economically. The classical strategies for the production of RNAs based on chemical synthesis or in vitro transcription are not viable due to their cost and yield. The most logical alternative is the production of dsRNA in biofactory systems such as bacterial cultures, for example, of *E. coli.*

The patent application WO 2015/177100 discloses the production of RNA by co-expressing a tRNA ligase and a chimeric RNA molecule comprising a target RNA and a plant viroid scaffold in a host cell. This co-expression causes the production of large amounts of the chimeric RNA molecule in the host cells and may be useful, for example, in the production of RNA aptamers and other RNA molecules. Recently, a system based on elements of the biology of viroids to produce large amounts of recombinant RNA in *E. coli* has been developed (Daros et al., 2018. Sci. Rep. 8: 1904).

Teresa Cordero et al. 2018 (Frontiers in Microbiology, vol.9, doi: 10.3389/fmicb.2018.00635) discloses the production of RNA using the ELVd system.

Feng Guo et al. 2002 (RNA, vol. 8, no. 5, pp. 647-658) describes an *in vivo* selection of *E. coli* mutants to improve the activity of the Tetrahymena pre-rRNA self-splicing intron in the context of heterologous exons. This document emphasizes the value of using libraries of random mutations to improve the activity of ribozymes in heterologous context *in vivo* (Abstract).

Roman et al. 1998 (Proceedings of National Academy of Sciences, vol. 95, no. 5, pp. 2134-2139) reports RNA-dependent integration of the Tetrahymena intron into the 23S rRNA in *E. coli* (Abstract). The study focuses on the contribution of reverse splicing to the spread of intron sequences during evolution and suggest ways in which self-splicing introns could be used as simple genetic vectors.

Viroids are a class of infectious agents of plants constituted exclusively by a noncoding circular RNA of relatively small size (246-401 nucleotides, in the known species to date). In spite of not coding own proteins, the viroid RNA manages to replicate itself and move around the plant, for which it has to recruit proteins and take advantage of the cellular structures of the host. The system of production of recombinant RNA in *E. coli* disclosed in Daros *et al.* 2018 (cited *ad supra)* is based on the co-expression of an RNA derived from the *Eggplant latent viroid* (ELVd), which contains the RNA of interest, and the chloroplastic isoform of the eggplant (*Solanum melongena* L.) tRNA ligase, an enzyme that mediates the circularization of this viroid in the infected plant. ELVd does not replicate in *E. coli.* However, when a precursor containing it is transcribed, its hammerhead ribozymes act to generate a linear monomer with the RNA of interest inserted. This monomer is recognized by the tRNA ligase and circularized. The resulting product, a hybrid molecule consisting of a highly structured viroid scaffold on which the RNA of interest is presented, is accumulated in enormous quantities in the bacterial cells as a consequence of its circularity and formation of a ribonucleoprotein complex with the tRNA ligase. With this system, quantities in the order of 100 mg of aptamers of RNA and other structured RNAs per litre of culture have been produced. However, due to the known instability of inverted DNA repeats in *E. coli,* this system was shown from the start to be incapable of producing substantial quantities of dsRNAs, the molecules necessary to induce gene silencing in pests and pathogens. Therefore, there is still the necessity of developing methods and processes that allow producing large amounts of dsRNA, overcoming the drawbacks of the systems, which already exist in the state of the art.

### SUMMARY OF THE INVENTION

Thanks to the use of autocatalytic introns in *E. coli,* the present invention allows to overcome the above-mentioned limitation of the state of the art, and to produce large quantities of dsRNAs which may be used against pests and pathogens. In addition, using these same autocatalytic introns permuted, it is possible to cleave the dsRNA of interest from the viroid scaffold on which it is transcribed, producing a pure circular dsRNA molecule without any residue of the viroid.

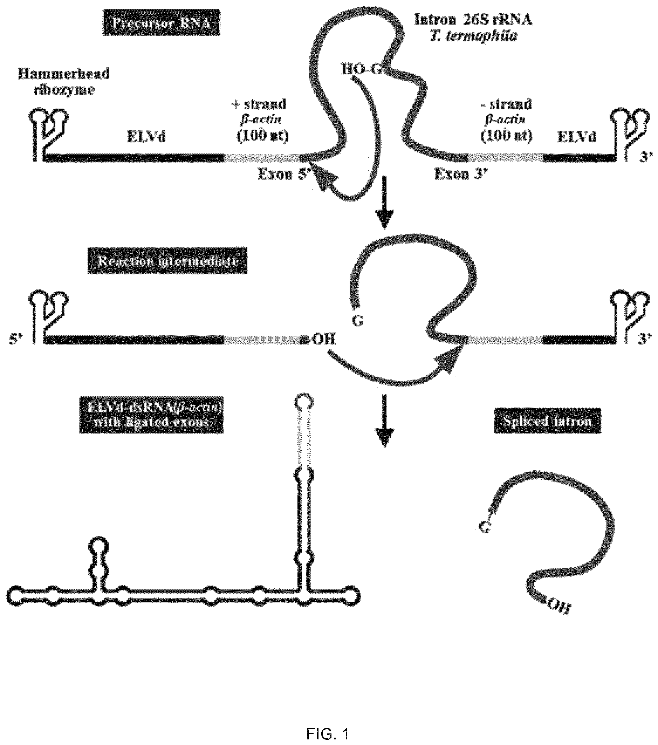

To solve the problem of the instability of inverted DNA repeats in *E. coli,* the repeats were separated with a cDNA corresponding to the group I intron of the 26S ribosomal RNA (rRNA) of prilled ciliated *Tetrahymena thermophila* (order Hymenostomatia). Since this intron is autocatalytic and only requires a guanosine nucleoside for its processing, once the transcript is expressed in *E. coli,* the intron is self-cleaved with extraordinary efficiency and a hairpin of dsRNA is formed closed by a loop of 20 nucleotides corresponding to two 10-nucleotides-long fragments of the exons that flank the intron. Thus, in the culture of *E. coli,* large quantities of hybrid circular molecules were produced efficiently consisting of an ELVd scaffold on which the pesticide dsRNA hairpin is presented (Figure 1).

Using this system, a 100 bp double-stranded RNA against essential genes of insect pests was produced and its pesticide activity demonstrated. However, it is understood that the commercial potential of this technology may be limited by the presence in the molecule of dsRNA of interest of a residue derived from the viroid.

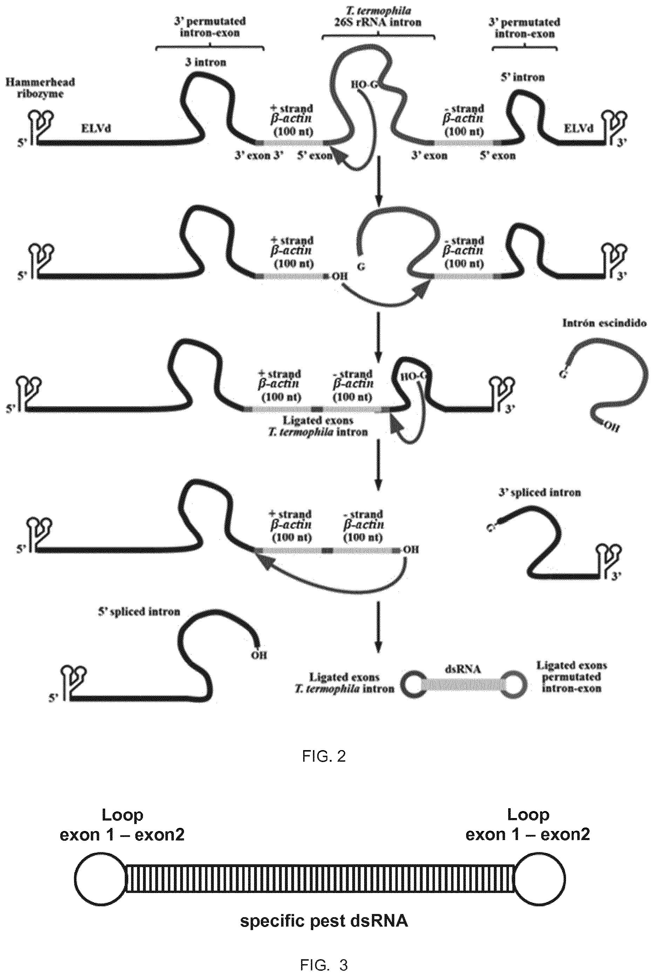

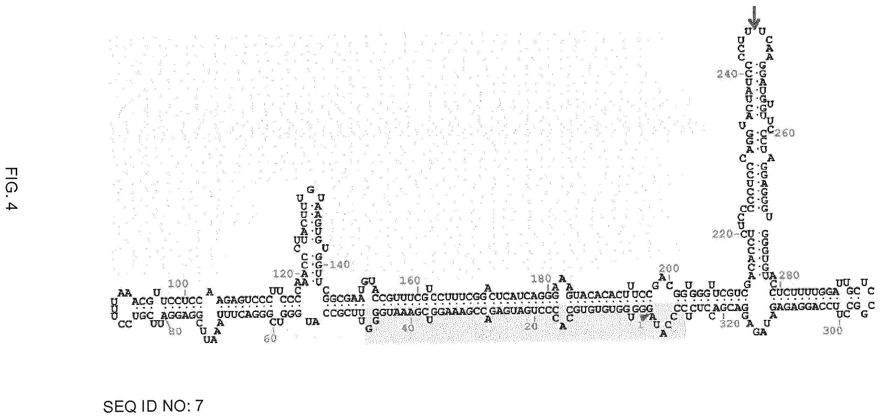

In order to eliminate the viroid moity, a new element consisting of a permuted intron-exon sequence was added to the system (Figure 2). When an intron of group I is divided in two parts, and the first is fused following the second, the self-cleavage reaction continues on the border between exon 1 and the 5' end of the intron and continues with the attack on 3' end from exon 1 to the border between the 3' end of the intron and exon 2, autocatalytically generating a circular RNA molecule formed by the two exons (Figure 3). In this way, the viroid moiety (Figure 4) is completely removed from the final dsRNA product (Figure 3).

In summary, this invention allows the production of large amounts of dsRNA in *E. coli.* To do this, the cDNAs of the two strands of the dsRNA are inserted into the cDNA of a Avsunviroidae, and separated by the cDNA of a type I intron. The co-expression in *E. coli* of the resulting RNA together with the tRNA ligase of eggplant produces a large accumulation of a hybrid molecule in which the dsRNA is fused to the viroid. The autocatalytic intron disappears during the processing of the precursor. In addition, if a permuted type I intron is added to this construct, the dsRNA of interest in a circular form can be separated from the viroid molecule.

In view of the foregoing, in an aspect, the present invention relates to an isolated nucleotide sequence comprising
a) the cDNAs of two strands of a target dsRNA separated by a type I autocatalytic intron flanked by exons or exon fragments, and
b) a plant Avsunviroidae sequence,
wherein element (a) is inserted in the plant Avsunviroidae sequence.

Additionally, in another inventive aspect, the present invention relates to a method for producing dsRNA specific for a target gene comprising co-expressing in a host cell
a) a nucleotide sequence according to the above-mentioned aspect, and
b) a nucleotide sequence encoding a tRNA ligase,
wherein the nucleotide sequences of a) and b) are in the same or different nucleic acid molecule.

### DETAILED DESCRIPTION OF THE INVENTION

In an aspect, the present invention relates to an isolated nucleotide sequence, hereinafter "nucleotide sequence of the invention", comprising
a) the cDNAs of two strands of a target dsRNA separated by a type I autocatalytic intron flanked by exons or exon fragments, and
b) a plant Avsunviroidae sequence,
wherein element (a) is inserted in the plant Avsunviroidae sequence.

As used herein, the term "double-stranded RNA" or "dsRNA" refers to a RNA molecule comprising two nucleic acid strands containing complementary sequences each other, wherein one of the strands is called sense strand (direction 5'-3') and the other one is called antisense strand (direction 3'-5'). Thus, the nucleotide sequence of the invention comprises an inverted repeat sequence comprising the sense and antisense strands of the target RNA separated by an intervening sequence comprising an intron type I flanked by exons or exon fragments.

As used herein, the term "target dsRNA" is the dsRNA capable of inducing the expression silencing of a specific target gene with which it shares homology. In the context of the present invention, it is considered that two nucleotide sequences share homology when the sequence identity between them is, at least, 75 % by the optimal alignment of them. Tools for determining the sequence identity between two nucleotide sequences are disclosed below. Any dsRNA molecule that needs to be generated in large amounts may be used as the target dsRNA. The target dsRNA may be nonnaturally occurring. Suitable target dsRNA may include sequences up to 100 bases, up to 200 bases or up to 300 bases or more. Preferably, the target dsRNA is not derived from a plant viroid and is not naturally associated with the plant viroid sequence (i.e. the target dsRNA is heterologous). Examples of specific target genes include, without being limited to, the target genes shown in Table 1.

**Table 1. Target genes useful for fighting against pests and pathogens**

| **Target insect** | **Target gene** | **Functions** |
|---|---|---|
| *Helicoverpa armigera* | *Cytochrome 450 monooxygenase CTP6AE14* | Degrade gossypol and increase tolerance to gossypol |
| | *GST glutathion transferase* | Detoxification enzymes |
| | *HaHR-3* | Molt-regulating transcription factor |
| | 20-Hydroxyecdysone | Role in molting and metamorphosis |
| | Arginine kinase | Play role in role in cellular energy metabolism |
| | Chitin synthase monooxygenase | Synthesis of chitin |
| | Cytochrome P450 | Required for tolerance against gossypol |
| | V-ATPase | Role in cellular energy production |
| | Chitinase | Role in molting and metamorphosis |
| *Diabrotica virgifera virgifera* LeConte | *Vacuolar ATPase* | Role in cellular energy production |
| | *DVSSJ1* | production of their respective smooth septate junction membrane proteins |
| | *DVSSJ2* | |
| *Spodoptera exigua* | 20-Hydroxyecdysone | Role in molting and metamorphosis |
| *Manduca sexta* | MsCYP6B46 | Nicotine degradation |
| *Nilaparvata Lugens* | Hexose transporter gene | Transport of glucose |
| | Carboxypeptidase | Hydrolysis of protein |
| | Trypsin like serine protease | Hydrolysis of protein |
| *Myzus persicae* | Rack1 | Essential in foraging and feeding of the pea aphid |
| | MpC002 | Intracellular receptor |
| | Hunchback (hb) | Play role in insect axial patterning |
| | Serine proteases (SP) | Gut proteolytic digestion |
| *Bemisia tabaci* | v-ATPase | Encode for v-ATPase subunit A, role in cellular energy production |
| | Acetylcholinesterase (AChE) | Required in signaling |
| | Ecdysone receptor (EcR) | Role in molting and metamorphosis |

In a particular embodiment, the target dsRNA is the gene beta-actin from *Ceratitis capitata* (diptera: *Tephritidae*). In a more particular embodiment, the cDNA sequence of the sense strand of the target dsRNA comprises, or consists of, a nucleotide sequence with a sequence identity of, at least, 70, 75, 80, 85, 90, 95, 96, 97, 98 or 99% with SEQ ID NO: 1. In a still more particular embodiment, the cDNA sequence of the sense strand of the target dsRNA comprises, or consists of, a nucleotide sequence with a sequence identity of 100% to SEQ ID NO: 1. In another particular embodiment, the cDNA sequence of the anti-sense strand of the target dsRNA comprises, or consists of, a nucleotide sequence with a sequence identity of, at least, 70, 75, 80, 85, 90, 95, 96, 97, 98 or 99% with SEQ ID NO: 2. In a still more particular embodiment, the cDNA sequence of the sense strand of the target dsRNA comprises, or consists of, a nucleotide sequence with a sequence identity of 100% to SEQ ID NO: 2.

SEQ ID NO: 1
GCTGACCGAAGCACCGTTGAACCCGAAGGCTAACCGCGAGAAGATGACCCAGATCATGTTCGAG
ACCTTCAACTCGCCGGCCATGTATGTGGCCATCCAG

SEQ ID NO: 2
CTGGATGGCCACATACATGGCCGGCGAGTTGAAGGTCTCGAACATGATCTGGGTCATCTTCTCG
CGGTTAGCCTTCGGGTTCAACGGTGCTTCGGTCAGC

As used herein, the term "sequence identity" refers to the degree of similarity between two nucleotide (or amino acid) sequences obtained by the optimal alignment of the two sequences. A degree of identity expressed as a percentage will be obtained based on the number of common residues among the aligned sequences. The degree of identity between two nucleotide (or amino acid) sequences can be determined by conventional methods, for example, standard sequence alignment algorithms known in the state of the art, such as BLAST [Altschul S.F. et al. Basic local alignment search tool. J Mol Biol. 1990 Oct 5; 215(3):403-10]. The BLAST programs, for example, BLASTN, BLASTX and TBLASTX, BLASTP and TBLASTN are a part of the public domain on The National Center for Biotechnology Information (NCBI) website. Particular nucleotide sequence variants may differ from a reference sequence by insertion, addition, substitution or deletion of 1 base, 2, 3, 4, 5-10, 10-20, 20-30 or more bases.

The cDNAs of two strands of a target dsRNA in the nucleotide sequence of the invention are separated by an autocatalytic intron flanked by exons or exon fragments. As used herein, the term "autocatalytic intron" refers to self-splicing introns which do not need the action of a spliceosome to be removed from the nucleotide sequence. There are three kinds of self-splicing introns, Type I, Type II and Type III.

Type I introns (or Group I) are large self-splicing ribozymes that catalyze their own excision from RNA precursors. They perform two sequential transesterification reactions. The core structure consists of nine paired regions (P1-P9). An exogenous guanosine or guanosine nucleotide (exoG) first docks onto the active G-binding site located in P7, and its 3'-OH is aligned to attack the phosphodiester bond at the 5' splice site located in P1. This results in a free 3'-OH group at the upstream exon and the exoG attached to the 5' end of the intron. Then, the terminal G (omega G) of the intron replaces the exoG and occupies the G-binding site to organize the second transesterification reaction. In this reaction, the 3'-OH group of the upstream exon in P1 is aligned to attack the 3' splice site in P10, leading to the ligation of the adjacent upstream and downstream exons and release of the catalytic intron. Examples of Type I introns include, without being limited to, autocatalytic intron type I of the rRNA 26S from *Tetrahymena thermophila, Ankistrodesmus stipitatus* LSU rRNA intron, *Saccharomyces cerevisiae* LSU rRNA mitochondrial intron or *Podospora anserina* mitochondrial apocytochrome b intron.

Type II introns (Group II) are a large class of self-splicing ribozymes and mobile genetic elements in which, unlike Group I introns, excision occurs in the absence of a guanosine nucleotide and a lariat structure is formed. The lariat contains an A-residue branchpoint what resembles that found in lariats formed during splicing of nuclear premRNA. The secondary structure of group II introns is characterized by six typical stemloop structures, domains I to VI (DI to DVI, or D1 to D6). The domains radiate from a central core that brings the 5' and 3' splice junctions into proximity. The proximal helix structures of the six domains are connected by a few nucleotides in the central region.

Type III introns (Group III) are a class of self-splicing ribozymes that have a conventional Group II-type DVI with a bulged adenosine, a streamlined DI, no DII-DV, and a relaxed splice site consensus. Splicing is done with two transesterification reactions with a DVI bulged adenosine as initiating nucleophile. The intron is also excised as a lariat.

In a more particular embodiment of the nucleotide sequence of the invention, the autocatalytic intron of type I is the autocatalytic intron type I of the rRNA 26S from *Tetrahymena thermophila* (GenBank access number V01416.1 [SEQ ID NO: 23]). It is also disclosed herein a fragment of the autocatalytic intron type I of the rRNA 26S from *Tetrahymena thermophila* with autocatalytic activity. The term "fragment of autocatalytic intron type I of the rRNA 26S from *Tetrahymena thermophila* with autocatalytic activity" means a nucleotide sequence having one or more (e.g., several) nucleotides absent from the 5' and/or 3' ends of a reference nucleotide sequence, wherein the fragment encodes a protein having the same activity than the reference nucleotide sequence, being thus a functionally equivalent fragment. In still more particular embodiment, the autocatalytic intron type I of the rRNA 26S from T. *thermophila* comprises, or consists of, a nucleotide sequence with a sequence identity of at least 70, 75, 80, 85, 90, 95, 96, 97, 98 or 99% with SEQ ID NO: 3. In a more particular embodiment, the autocatalytic intron comprises, or consists of, a nucleotide sequence with a sequence identity of 100% to SEQ ID NO: 3.

SEQ ID NO: 3
AAATAGCAATATTTACCTTTGGAGGGAAAAGTTATCAGGCATGCACCTGGTAGCTAGTCTTTAAACCAAT
AGATTGCATCGGTTTAAAAGGCAAGACCGTCAAATTGCGGGAAAGGGGTCAACAGCCGTTCAGTACCAAG
TCTCAGGGGAAACTTTGAGATGGCCTTGCAAAGGGTATGGTAATAAGCTGACGGACATGGTCCTAACCAC
GCAGCCAAGTCCTAAGTCAACAGATCTTCTGTTGATATGGATGCAGTTCACAGACTAAATGTCGGTCGGG
GAAGATGTATTCTTCTCATAAGATATAGTCGGACCTCTCCTTAATGGGAGCTAGCGGATGAAGTGATGCA
ACACTGGAGCCGCTGGGAACTAATTTGTATGCGAAAGTATATTGATTAGTTTTGGAGTACTCG

In the present invention, the autocatalytic intron is flanked by exons or exon fragments, which may proceed from the same or different microorganism than the autocatalytic intron. The term "fragment" has been defined above. In a particular embodiment, the autocatalytic intron and the exons (or exon fragments) proceed from the same microorganism, particularly, said microorganism is *T. thermophila.* Likewise, the exons (or exon fragments) in 5' and 3' position with respect to the autocatalytic intron may proceed from the same or different microorganism. In a more particular embodiment, the exon (or exon fragment) at 5' position of the autocatalytic intron comprises, or consists of, the sequence SEQ ID NO: 4 and/or the exon (or exon fragment) at 3' position of the autocatalytic intron comprises, or consists of, the sequence SEQ ID NO: 5.
SEQ ID NO: 4 - **ATGACTCTCT**
SEQ ID NO: 5 - **TAAGGTAGCC**

Thus, in a more particular embodiment, the autocatalytic intron flanked by exons (or exon fragments) comprises, or consisting of, the sequence SEQ ID NO: 6, wherein the exons are shown below underlined.

SEQ ID NO: 6
ATGACTCTCTAAATAGCAATATTTACCTTTGGAGGGAAAAGTTATCAGGCATGCACCTGGTAGCTAGTCT
TTAAACCAATAGATTGCATCGGTTTAAAAGGCAAGACCGTCAAATTGCGGGAAAGGGGTCAACAGCCGTT
CAGTACCAAGTCTCAGGGGAAACTTTGAGATGGCCTTGCAAAGGGTATGGTAATAAGCTGACGGACATGG
TCCTAACCACGCAGCCAAGTCCTAAGTCAACAGATCTTCTGTTGATATGGATGCAGTTCACAGACTAAAT
GTCGGTCGGGGAAGATGTATTCTTCTCATAAGATATAGTCGGACCTCTCCTTAATGGGAGCTAGCGGATG
AAGTGATGCAACACTGGAGCCGCTGGGAACTAATTTGTATGCGAAAGTATATTGATTAGTTTTGGAGTAC
TCGTAAGGTAGCC

The nucleotide sequence of the invention also comprises a plant Avsunviroidae sequence. The plant viroid sequence is deemed to be the scaffold of the nucleotide sequence of the invention on which the rest of the components (the cDNAs of two strands of a dsRNA separated by an autocatalytic intron flanked by exons) are inserted. As used herein, the term "plant viroid" refers to the smallest infectious pathogens known comprising solely of a short strand of circular, single-stranded RNA that has no protein coating. The viroid replication mechanism uses the host cell enzyme RNA polymerase II or chloroplastic RNA polymerase, which catalyze "rolling circle" synthesis of new RNA using the viroid RNA as a template. Taxonomically, plant viroids are divided in two families: Family *Pospiviroidae* comprising the genera *Pospiviroid, Hostuviroid, Cocadviroid, Apscaviroid* and *Coleviroid,* and family *Avsunviroidae* comprising the genera *Avsunviroid, Pelamoviroid* and *Elaviroid.* Examples of viroids include, without being limited to, *Potato spindle tuber viroid* (PSTVd), *Citrus exocortis viroid* (CEVd), *Chrysanthemum chlorotic mottle viroid* (CChMVd), *Hop stunt viroid* (HSVd), *Peach latent mosaic viroid* (PLMVd), *Coconut cadang-cadang viroid* (CCCVd), *Apple scar skin viroid* (ASSVd), *Coleus blumei viroid 1* (CbVd-1), *Avocado sunblotch viroid* (ASBVd), *Peach latent mosaic viroid* (PLMVd), and *Eggplant latent viroid* (ELVd). In case that the plant viroid sequence used does not comprise a hammerhead ribozyme domain, i.e. it belongs to the Family *Pospiviroidae,* the enzymes responsible for strand scission of the viroids are co-expressed together with the plant viroid or the nucleotide sequence of the invention is expressed in a host cell comprising said enzymes. When the plant viroid sequence used in the present invention comprises a hammerhead domain, i.e. belongs to the family *Avsunviroidae,* two functional hammerhead domains, one to each side of the plant viroid sequence, have to be added to the plant viroid sequence, duplicating the hammerhead domain of the plant viroid. In a particular embodiment of the present invention, the plant Avunviroidae sequence is the ELVd sequence, the ASBVd sequence, the PLMVd sequence, or the CChMVd sequence.

The plant viroid sequence may comprise all or part of a plant viroid. The full-length (333 nt) genomic sequence of ELVd is publically available under the GenBank entry number AJ536613.1 GI: 29825431 (SEQ ID NO: 7). The sequence and structure of ELVd is shown in Figure 4. The hammerhead ribozyme region of ELVd corresponds to nucleotides 327 to 46 (plus) and nucleotides 153-203 (minus).

SEQ ID NO: 7

1 GGGTGGTGTG TGCCACCCCT GATGAGACCG AAAGGTCGAA ATGGGGTTTC GCCATGGGTC

61 GGGACTTTAA ATTCGGAGGA TTCGTCCTTT AAACGTTCCT CCAAGAGTCC CTTCCCCAAA

121 CCCTTACTTT GTAAGTGTGG TTCGGCGAAT GTACCGTTTC GTCCTTTCGG ACTCATCAGG

181 GAAAGTACAC ACTTTCCGAC GGTGGGTTCG TCGACACCTC TCCCCCTCCC AGGTACTATC

241 CCCTTTCAAG GATGTGTTCC CTAGGAGGGT GGGTGTACCT CTTTTGGATT GCTCCGGCCT

301 TCCAGGAGAG ATAGAGGACG ACCTCTCCCC ATA

The full-length (247 nt) genomic sequence of ASBVd is publically available under the GenBank entry number NC_001410.1 GI: 11496574 (SEQ ID NO: 8).

SEQ ID NO: 8

1 TTTATTAGAA CAAGAAGTGA GGATATGATT AAACTTTGTT TGACGAAACC AGGTCTGTTC
61 CGACTTTCCG ACTCTGAGTT TCGACTTGTG AGAGAAGGAG GAGTCGTGGT GAACTTTTAT
121 TAAAAAAATT AGTTCACTCG TCTTCAATCT CTTGATCACT TCGTCTCTTC AGGGAAAGAT
181 GGGAAGAACA CTGATGAGTC TCGCAAGGTT TACTCCTCTA TCTTCATTGT TTTTTTACAA
241 AATCTTG

The full-length (399 nt) genomic sequence of CChMVd is publically available under the GenBank entry number NC_003540.1 GI: 20095240 (SEQ ID NO: 9).

SEQ ID NO: 9

1 GGCACCTGAC GTCGGTGTCC TGATGAAGAT CCATGACAGG ATCGAAACCT CTTCCAGTTT
61 CGGCTTGTGT GGGAGTAAAG CTTTCGCTCT CTCCACAGCC TCATCAGGAA ACCCACTTCA
121 GGTCTCGACT GGAAGGTCGT TAAACTTCCC CTCTAAGCGG AGTAGAGGTA AATACCTCCG
181 TCCAACCCCG GGAGGAAAGG GGTTGGGACC CGGAACAGAT CCAGTTCCGG TCCTTTGGAG
241 TCCATTTCTC TCGTTGGATA TTCTCCTCGG AGAAGGGAGA TGGGGCCAGT CCCAGTCGGT
301 TCGCTCTCGT AGTCACAGCC ACTGGGGAAC CTAGGCAGAT GGCTGGACGG AGTCTTAGTC
361 CACTCCAGAG GACCTTGGGT TTGAAACCCC CAAGAGGTC

The full-length (337 nt) genomic sequence of PLMVd is publically available under the GenBank entry number NC_003636.1 GI: 20177433 (SEQ ID NO: 10). The hammerhead ribozyme region of PLMVd corresponds to nucleotides 282 to 335 (plus) and nucleotides 2-57 (minus).

SEQ ID NO: 10

1 GTCATAAGTT TCGTCGCATT TCAGCGACTC ATCAGTGGGC TTAGCCCAGA CTTATGAGAG
61 AGTAAAGACC TCTCAGCCCC TCCACCTTGG GGTGCCCTAT TCGGAGCACT GCAGTTCCCG
121 ATAGAAAGGC TAAGCACCTC GCAATGAGGT AAGGTGGGAC TTTTCCTTCT GGAACCAAGC
181 GGTTGGTTCC GAGGGGGGTG TGATCCAGGT ACCGCCGTAG AAACTGGATT ACGACGTCTA
241 CCCGGGATTC AAACCCGTCC CCTCCAGAAG TGATTCTGGA TGAAGAGTCT GTGCTAAGCA
301 CACTGACGAG TCTCTGAGAT GAGACGAAAC TCTTCTT

A variant of a reference plant viroid sequence listed above (for example, the genomic sequence of ELVd, ASBVd, CChMVd or PLMVd, (SEQ ID NO: 7 to 10 respectively); or a truncated form thereof, as set out above) may comprise, or consist of, a nucleotide sequence having at least 70, 75, 80, 85, 90, 95, 96, 97, 98, 99% sequence identity to the sequence of the reference plant viroid scaffold.

The plant Avsunviroidae sequence may be a truncation of the full-length plant viroid sequence, for example the full-length plant viroid genome sequence of any of SEQ ID NO: 7 to 10. The plant viroid sequence may comprise, or consist of, the full-length plant viroid sequence with one or more deletions. The plant viroid sequence may be a synthetic plant viroid sequence that does not occur in nature.

In a more particular embodiment, the plant viroid sequence comprises the *Eggplant latent viroid* sequence (ELVd) which, in a still more particular embodiment, comprises, or consists of, a nucleotide sequence with a sequence identity of, at least, 70, 75, 80, 85, 90, 95, 96, 97, 98 or 99% with SEQ ID NO: 11. In another particular embodiment, the ELVd comprises, or consists of, a nucleotide sequence with a sequence identity of 100% with SEQ ID NO: 11.

SEQ ID NO: 11
CCCCATAGGGTGGTGTGTGCCACCCCTGATGAGACCGAAAGGTCGAAATGGGGTTTCGCCATGGGTCGGG
ACTTTAAATTCGGAGGATTCGTCCTTTAAACGTTCCTCCAAGAGTCCCTTCCCCAAACCCTTACTTTGTA
AGTGTGGTTCGGCGAATGTACCGTTTCGTCCTTTCGGACTCATCAGGGAAAGTACACACTTTCCGACGGT
GGGTTCGTCGACACCTCTCCCCCTCCCAGGTACTATCCCCTTTCAAGGATGTGTTCCCTAGGAGGGTGGG
TGTACCTCTTTTGGATTGCTCCGGCCTTCCAGGAGAGATAGAGGACGACCTCTCCCCATAGGGTGGTGTG
TGCCACCCCTGATGAGACCGAAAGGTCGAAATGGGG

Underlined: the hammerhead ribozyme sequences

Double underlined: insertion site of the inverted repeat sequence comprising the sense and antisense strands of the target RNA separated by an intervening sequence comprising an intron type I flanked by exons or exon fragments.

**Bold:** cut site of the ribozyme
In a particular embodiment of the nucleotide sequence of the invention, the plant Avsunviroidae comprises a hammerhead ribozyme domain (motif), or a fragment thereof, at the 5' and 3' end of the sequence of the plant viroid. As used herein, the term "hammerhead ribozymes domain" is a small self-cleaving RNA that promotes strand scission by internal phosphoester transfer. The term "fragment" has been defined above. Suitable plant viroid sequences may comprise the hammerhead ribozyme region of the plant viroid, which may for example be located from nucleotides 327 to 46 (plus) and nucleotides 153-203 (minus) in ELVd and at the corresponding nucleotides in other plant viroids. Corresponding nucleotides in other plant viroid genomes may be identified using standard sequence alignment tools. In a particular embodiment of the present invention, the hammerhead ribozyme domain located at the 5' end comprises nucleotides 1 to 53 of SEQ ID NO: 11, and/or the hammerhead ribozyme domain located at the 3' end comprises nucleotides 334 to 386 of SEQ ID NO: 11.

The inverted repeat sequence, comprising the sense and antisense strands of the target RNA separated by an intervening sequence comprising an autocatalytic type I intron flanked by exons or exon fragments, may be located at any position within the plant viroid scaffold as long as it does not disrupt the viroid activity. Thus, the inverted repeat sequence may be located in any site between the viroid hammerhead ribozyme domains of the plant Avsunviroidae sequence. In a more particular embodiment, the insertion site of the inverted repeat sequence is located between nucleotides 252 and 253 of SEQ ID NO: 11.

In a still more particular embodiment, the nucleotide sequence of the invention comprises, or consists of, the sequence SEQ ID NO: 12.

SEQ ID NO: 12
CCCCATAGGGTGGTGTGTGCCACCCCTGATGAGACCGAAAGGTCGAAATGGGGTTTCGCCATGGGTCGGG
ACTTTAAATTCGGAGGATTCGTCCTTTAAACGTTCCTCCAAGAGTCCCTTCCCCAAACCCTTACTTTGTA
AGTGTGGTTCGGCGAATGTACCGTTTCGTCCTTTCGGACTCATCAGGGAAAGTACACACTTTCCGACGGT
GGGTTCGTCGACACCTCTCCCCCTCCCAGGTACTATCCCCTTGCTGACCGAAGCACCGTTGAACCCGAAG
GCTAACCGCGAGAAGATGACCCAGATCATGTTCGAGACCTTCAACTCGCCGGCCATGTATGTGGCCATCC
AGATGACTCTCTAAATAGCAATATTTACCTTTGGAGGGAAAAGTTATCAGGCATGCACCTGGTAGCTAGT
CTTTAAACCAATAGATTGCATCGGTTTAAAAGGCAAGACCGTCAAATTGCGGGAAAGGGGTCAACAGCCG
TTCAGTACCAAGTCTCAGGGGAAACTTTGAGATGGCCTTGCAAAGGGTATGGTAATAAGCTGACGGACAT
GGTCCTAACCACGCAGCCAAGTCCTAAGTCAACAGATCTTCTGTTGATATGGATGCAGTTCACAGACTAA
ATGTCGGTCGGGGAAGATGTATTCTTCTCATAAGATATAGTCGGACCTCTCCTTAATGGGAGCTAGCGGA
TGAAGTGATGCAACACTGGAGCCGCTGGGAACTAATTTGTATGCGAAAGTATATTGATTAGTTTTGGAGT
ACTCGTAAGGTAGCCCTGGATGGCCACATACATGGCCGGCGAGTTGAAGGTCTCGAACATGATCTGGGTC
ATCTTCTCGCGGTTAGCCTTCGGGTTCAACGGTGCTTCGGTCAGCTCAAGGATGTGTTCCCTAGGAGGGT
GGGTGTACCTCTTTTGGATTGCTCCGGCCTTCCAGGAGAGATAGAGGACGACCTCTCCCCATAGGGTGGT
GTGTGCCACCCCTGATGAGACCGAAAGGTCGAAATGGGG

Underlined: the hammerhead ribozyme sequences showing in **bold the** cut site of the ribozyme.

Broken underlined: cDNA from ELVd (C327 to G46 from GeneBank accession number AJ536613.1; SEQ ID NO: 7), showing in double underlined the insertion site of the inverted repeat sequence comprising the sense and antisense strands of the target RNA separated by an intervening sequence comprising a type I intron flanked by exons.

*Italic:* 100 bp of the β-actin gene of *Ceratitis capitata,* in sense and antisense orientation.

Undulating underlined: 5' and 3' exon fragments of the autocatalytic type I intron of the rRNA 26S gene from *T. thermophila.*

Underlined with dots: Autocatalytic type I intron of the rRNA 26S gene from *T*. *thermophila.*

The nucleotide sequence of the invention as disclosed in previous paragraphs allows the skilled person in the art to produce large amounts of dsRNA when it is cloned in a host cell, such as, for example, *E. coli,* along with a tRNA ligase. Thus, co-expression of the nucleotide sequence of the invention with tRNA ligase is shown herein to lead to the high level production of dsRNA molecule in host cells. As the skilled person knows, the tRNA ligase is needed to catalyze the covalent joining of single-stranded 5'-hydroxyl termini of RNA to single-stranded 2'-3'-cyclic phosphodiester termini of RNA, and in this way to form the circular RNA that contains the dsRNA of interest. The tRNA ligase may be bound to the nucleotide sequence of the invention giving rise to a chimeric complex, or may be present in the vector used for cloning the nucleotide sequence in the host cell, or may be present in the host cell. In a particular embodiment, the nucleotide sequence of the invention further comprises a nucleotide sequence encoding a tRNA ligase.

The tRNA ligase may be a plant tRNA ligase, for example a plant choloroplast or plant nuclear tRNA ligase. Preferably, the tRNA ligase is a plant chloroplast tRNA ligase. The tRNA ligase may be from a eukaryote other than a plant. Suitable tRNA ligases may be homologues or orthologues of plant tRNA ligases and may be readily identified using standard techniques. In some embodiments, suitable tRNA ligases may bind to the plant viroid sequence to form a ribonucleoprotein complex. In some preferred embodiments, the plant viroid sequence and the tRNA ligase may be from the same plant species.

Suitable plant tRNA ligases are well-known in the art and include, without being limited to, *Solanum melongena* (eggplant) tRNA ligase. The amino acid sequence of *Solanum melongena* tRNA ligase is publicly available under the GenBank entry number AFK76482.1 GI: 388604525 (SEQ ID NO: 13) and the nucleotide coding sequence is publicly available under the GenBank entry number JX025157.1 GI: 388604524 (SEQ ID NO: 14).
SEQ ID NO. 13 
1   MSVSHRVIYS FTHYKLYNLS SSLSSLPSRI FFPFQSPSFH TFSSLMPNNQ ERGGYEGKKW
61  QVRPSSNRVP GSSSNVEPVS AATAEAITDR LKSVDITESG AQSSVPVTSL QFGSVGLAPQ
121 SPVQHQKVIW KPKSYGTVSG APVVEAGKTP VEQKSALLSK LFKGNLLENF TVDNSTFSRA
181 QVRATFYPKF ENEKSDQEIR TRMIEMVSKG LAIVEVTLKH SGSLFMYAGH EGGAYAKNSF
241 GNIYTAVGVF VLGRMFREAW GTKASKKQAE FNEFLERNRM CISMELVTAV LGDHGQRPRD
301 DYAVVTAVTE LGNGKPTFYS TPDVIAFCRE WRLPTNHVWL FSTRKSVTSF FAAYDALCEE
361 GTATTVCEAL SEVADISVPG SKDHIKVQGE ILEGLVARIV KRESSEHMER VLRDFPPPPS
421 EGEGLDLGPT LREICAANRS EKQQIKALLQ SAGTAFCPNY LDWFGDENSG SHSRNADRSV
481 VSKFLQSHPA DLYTGKIQEM VRLMREKRFP AAFKCHYNLH KINDVSSNNL PFKMVIHVYS
541 DSGFRRYQKE MRHKPGLWPL YRGFFVDLDL FKVNEKKTAE MAGSNNQMVK NVEEDNSLAD
601 EDANLMVKMK FLTYKLRTFL IRNGLSTLFK EGPSAYKSYY LRQMKIWNTS AAKQRELSKM
661 LDEWAVYIRR KYGNKPLSSS TYLSEAEPFL EQYAKRSPQN HALIGSAGNF VKVEDFMAIV
721 EGEDEEGDLE PAKDIAPSSP SISTRDMVAK NEGLIIFFPG IPGCAKSALC KEILNAPGGL
781 GDDRPVNSLM GDLIKGRYWQ KVADERRRKP YSIMLADKNA PNEEVWKQIE NMCLSTGASA
841 IPVIPDSEGT ETNPFSIDAL AVFIFRVLHR VNHPGNLDKS SPNAGYVMLM FYHLYDGKSR
901 QEFESELIER FGSLVRIPVL KPERSPLPDS VRSIIEEGLS LYRLHTTKHG RLESTKGTYV
961 QEWVKWEKQL RDILLGNADY LNSIQVPFEF AVKEVLEQLK VIARGEYAVP AEKRKLGSIV
1021 FAAISLPVPE ILGLLNDLAK KDPKVGDFIK DKSMESSIQK AHLTLAHKRS HGVTAVANYG
1081 SFLHQKVPVD VAALLFSDKL AALEAEPGSV EGEKINSKNS WPHITLWSGA GVAAKDANTL
1141 PQLLSQGKAT RIDINPPVTI TGTLEFF
SEQ ID NO. 14

1 GTGACTGGCT CAAAGGCGGT AGTAGTGGCT CTTCTTCCCC GCTGGAATTT CTCGCCTATT
61 TTATATTGAT TGACAGCAGC CGAGTCTTCC TTTTGTGTAG AACTGAATGT CGGTTTCGCA
121 TAGGGTCATT TACTCTTTCA CTCATTACAA ACTCTATAAT CTCTCTTCTT CTTTATCATC
181 TTTGCCTTCT AGAATCTTCT TCCCTTTTCA ATCTCCTTCC TTTCACACGT TCTCTTCACT
241 CATGCCCAAC AATCAGGAAA GGGGTGGTTA TGAAGGAAAA AAATGGCAAG TGAGGCCAAG
301 TTCCAATAGG GTACCAGGCT CGTCTTCAAA TGTGGAACCT GTATCTGCTG CAACTGCTGA
361 AGCCATTACC GACCGTCTAA AGTCCGTGGA TATTACTGAA AGTGGTGCAC AGTCTAGTGT
421 TCCAGTCACA TCTCTTCAGT TTGGCAGCGT TGGATTAGCA CCCCAGTCAC CTGTGCAACA
481 TCAAAAAGTA ATCTGGAAAC CCAAATCATA TGGAACAGTG TCTGGAGCCC CAGTGGTTGA
541 AGCTGGAAAA ACACCAGTTG AACAAAAAAG TGCTCTTTTA AGTAAATTAT TCAAGGGTAA
601 TTTATTGGAA AATTTTACTG TAGATAACTC AACATTCTCG AGAGCCCAAG TAAGGGCCAC
661 TTTCTACCCA AAATTTGAGA ATGAGAAATC AGATCAGGAG ATCAGGACAA GGATGATAGA
721 GATGGTCTCC AAAGGCTTGG CTATAGTCGA GGTCACACTT AAGCATTCTG GATCTCTTTT
781 TATGTATGCT GGGCATGAAG GTGGAGCATA TGCCAAGAAT AGCTTCGGGA ATATCTATAC
841 TGCCGTTGGC GTCTTTGTTC TTGGACGGAT GTTTCGTGAG GCATGGGGAA CTAAAGCAAG

901 CAAGAAGCAA GCAGAGTTCA ATGAGTTTCT TGAGCGCAAT CGTATGTGCA TATCAATGGA
961 GTTGGTCACG GCAGTGTTGG GGGACCACGG ACAACGCCCA CGAGATGATT ATGCGGTTGT
1021 GACTGCAGTC ACGGAGTTGG GAAATGGAAA ACCAACTTTC TATTCAACTC CCGATGTAAT
1081 TGCTTTTTGC AGGGAATGGC GATTACCAAC AAATCATGTA TGGCTGTTCT CAACAAGGAA
1141 ATCAGTGACT TCCTTCTTTG CTGCGTATGA TGCACTTTGC GAGGAAGGTA CAGCAACCAC
1201 CGTTTGCGAG GCTCTCAGCG AAGTTGCTGA TATTTCTGTA CCTGGATCAA AAGACCATAT
1261 AAAAGTGCAG GGTGAAATTT TGGAGGGTCT CGTGGCCCGC ATCGTAAAAC GTGAGAGCTC
1321 AGAGCATATG GAGCGGGTTC TGAGAGATTT TCCTCCTCCG CCATCAGAGG GTGAGGGTTT
1381 GGACCTGGGA CCTACGTTAC GTGAAATTTG TGCTGCAAAC AGATCAGAAA AGCAGCAAAT
1441 AAAGGCACTT CTTCAGAGTG CTGGCACGGC TTTCTGCCCG AATTATTTGG ACTGGTTTGG
1501 AGATGAAAAC TCTGGTTCAC ATTCAAGAAA TGCTGATCGA TCTGTTGTCT CAAAGTTCTT
1561 ACAATCACAT CCTGCTGATC TTTATACAGG AAAAATACAG GAAATGGTTC GCTTGATGAG
1621 GGAAAAGCGC TTTCCTGCTG CTTTCAAGTG TCATTATAAC TTACATAAAA TTAATGATGT
1681 ATCGAGTAAC AACCTGCCTT TCAAAATGGT GATCCATGTA TATAGTGATT CAGGCTTCCG
1741 CCGGTACCAG AAAGAGATGA GGCACAAACC AGGACTATGG CCTTTGTATC GAGGCTTTTT
1801 TGTTGACCTG GATTTATTCA AGGTCAATGA GAAGAAAACT GCTGAAATGG CAGGAAGCAA
1861 CAATCAAATG GTAAAAAATG TGGAAGAGGA CAACAGTTTA GCTGATGAAG ATGCAAATCT
1921 GATGGTCAAG ATGAAATTTC TTACTTACAA GTTGAGAACT TTTTTGATCC GTAATGGCTT
1981 GTCGACTCTT TTCAAAGAAG GACCTTCTGC GTATAAGTCT TATTACCTGA GGCAAATGAA
2041 AATTTGGAAT ACTTCAGCAG CCAAGCAACG AGAACTCAGC AAGATGCTTG ATGAATGGGC
2101 AGTATATATA CGCAGAAAAT ATGGGAACAA ACCATTGTCA TCATCCACAT ACCTAAGTGA
2161 AGCTGAGCCT TTCCTTGAAC AATATGCAAA GCGTAGTCCA CAAAATCATG CTTTGATAGG
2221 ATCTGCTGGA AATTTTGTCA AAGTTGAAGA TTTCATGGCT ATTGTTGAAG GAGAAGATGA
2281 AGAGGGTGAT CTCGAGCCTG CGAAAGATAT TGCTCCTTCA AGCCCTAGTA TTTCCACCAG
2341 AGACATGGTG GCAAAGAATG AGGGTCTCAT TATTTTCTTT CCAGGAATAC CAGGTTGTGC
2401 TAAATCTGCA CTTTGTAAGG AAATACTGAA TGCTCCAGGA GGGCTTGGAG ATGATCGACC
2461 AGTTAACAGT TTAATGGGTG ATCTTATTAA AGGTAGATAT TGGCAAAAAG TTGCTGATGA
2521 ACGTCGAAGA AAACCTTACT CGATCATGCT TGCTGACAAG AATGCACCAA ATGAGGAAGT
2581 ATGGAAACAA ATTGAGAACA TGTGCCTAAG CACCGGAGCA TCTGCTATTC CAGTTATACC
2641 TGATTCAGAA GGAACTGAAA CTAATCCATT CTCTATTGAT GCACTTGCGG TTTTTATATT
2701 CCGAGTACTT CACCGTGTCA ATCATCCGGG AAATCTTGAC AAGTCATCTC CAAATGCTGG
2761 ATATGTGATG CTTATGTTTT ATCACCTTTA TGATGGAAAG AGCCGTCAGG AGTTCGAGAG
2821 TGAGCTTATT GAACGTTTTG GATCGCTTGT CAGAATTCCT GTACTGAAAC CTGAGAGGTC
2881 TCCTCTTCCG GATTCTGTGA GGTCTATTAT CGAGGAGGGA CTCAGTCTGT ACAGACTTCA
2941 TACAACGAAA CATGGAAGAT TGGAGTCTAC AAAAGGGACA TATGTACAAG AGTGGGTTAA
3001 ATGGGAGAAG CAATTGAGAG ATATTCTACT TGGAAATGCA GACTATCTCA ATTCAATACA
3061 GGTTCCATTT GAATTTGCCG TTAAAGAAGT CCTTGAACAA CTGAAAGTTA TTGCGAGGGG
3121 CGAATATGCA GTGCCTGCTG AGAAGAGGAA GCTAGGATCC ATTGTATTCG CCGCTATCAG
3181 CCTGCCAGTT CCAGAAATTC TAGGTCTTCT AAATGATCTA GCAAAGAAAG ATCCAAAGGT
3241 TGGCGATTTC ATTAAGGACA AGAGCATGGA GAGCAGCATT CAGAAGGCCC ATCTTACCCT
3301 GGCTCACAAG AGAAGTCACG GTGTCACTGC AGTTGCCAAT TACGGTTCCT TTCTTCATCA
3361 AAAGGTGCCA GTAGACGTGG CTGCTTTGTT GTTCTCCGAT AAATTGGCTG CACTAGAAGC
3421 TGAGCCTGGC TCTGTTGAAG GTGAAAAGAT CAATTCTAAA AACTCATGGC CCCATATCAC

3481 ATTATGGTCT GGTGCAGGAG TTGCCGCAAA AGATGCCAAT ACACTACCAC AGTTACTTTC
3541 CCAAGGGAAG GCTACCCGCA TTGATATAAA TCCACCGGTC ACTATAACTG GCACTCTCGA
3601 ATTCTTTTGA ACTCTACTTG TAATTCCTCT TGTGTGGATT TACATAATGT GAACCCTACA
3661 TGGGTTCTGA GTGAATCATG TAATGATTCT CCTGAGTTTT AGGAACAATT TTGTAGCAGT
3721 CTTGCAGTTG CAGGATAGAA CTAGATCTAG AGAAAATGTA AAGATGTTTA AGGCAATAAA
3781 GGTCTTGAGC TGTGGGCATA TTGAAAATCC CCAGTAGCAT TTTTCTTGAG CAAAAAAAAA
3841 AAAAAAAA

Thus, in a particular embodiment of the present invention, the tRNA ligase is the eggplant tRNA ligase. In a more particular embodiment, the tRNA ligase from eggplant comprises, or consists of, a nucleotide sequence having a sequence identity of at least 70, 75, 80, 85, 90, 96, 97, 98 or 99% to SEQ ID NO: 14. In a more particular embodiment, the tRNA ligase from eggplant comprises, or consists of, a nucleotide sequence having a sequence identity of 100% to SEQ ID NO: 14.

Other suitable tRNA ligases for use in the present invention may be an orthologue from a plant species other than *Solanum melongena* (eggplant), for example *Arabidopsis thaliana* (GenBank accession number NM_100665.3 [SEQ ID NO: 18]), *Solanum lycopersicum* (XM_004251213.4 [SEQ ID NO: 19]), *Hevea brasiliensis* (XM_021784835.1 [SEQ ID NO: 20]), *Prunus avium* (XM_021975205.1 [SEQ ID NO: *21]), Malus domestica* (XM_029088496.1 [SEQ ID NO: 22]), *Triticum* spp. or *Oryza sativa.* Suitable orthologues may be identified using standard sequence analysis techniques.

In a particular embodiment, the plant viroid and the tRNA ligase may be from the same plant species. For example, eggplant tRNA ligase may be expressed with an ELVd sequence, or avocado tRNA ligase may be expressed with an *Avocado sunblotch viroid* (ASBVd) sequence. The dsRNA molecule and the tRNA ligase may be encoded by the same or different nucleic acids in the host cell. The nucleic acids may be heterologous or exogenous to the host cell and may be introduced into the host cell by transformation or transfection as described below.

As a consequence of expressing the nucleotide sequence of the invention on a host cell in combination with a tRNA ligase, large quantities of hybrid circular molecules are produced efficiently comprising the plant viroid on which the dsRNA hairpin is presented. For example, more than 10 mg, more than 20 mg, more than 30 mg, more than 50 mg or more than 100 mg of dsRNA may be produced per litre of the host cell culture. The data herein, for example, shows the production of 50 mg per litre of *E. coli* culture.

However, it is desirable to avoid the presence of the plant viroid in the dsRNA molecule in order to improve the commercial potential of this technology. In order to remove the residue derived from the plant viroid, the inventors added a new element to the nucleotide sequence of the invention comprising of a permuted type I intron-exon sequence. When an intron of group I is divided into two parts, and the first is fused following the second, the self-cleavage reaction continues on the border between exon 1 and the 5' end of the intron and continues with the attack on 3' end from exon 1 to the border between the 3' end of the intron and exon 2, autocatalytically generating a circular RNA molecule comprised by the two exons.

Therefore, in another particular embodiment, the nucleotide sequence of the invention may further comprise permuted type I autocatalytic intron-exon sequences between the plant viroid sequence and the cDNAs sequences. Examples of permuted autocatalytic intron-exon sequences include, without being limited to, tRNA intron from *Anabaena* sp. strain PCC7120 or the intron from thymidylate synthase (td) gene of phage T4. In a particular embodiment, the permuted type I autocatalytic intron-exon sequences are the autocatalytic type I intron of the 26S rRNA gene of *T. thermophila.*

In a more particular embodiment, the permuted type I autocatalytic intron-exon sequence at 5' of the cDNA sequence of the sense strand of the target dsRNA comprises, or consists of, a nucleotide sequence with a sequence identity of at least 70, 75, 80, 85, 90, 95, 96, 97, 98, 99% with SEQ ID NO: 15. In a still more particular embodiment, the permuted type I autocatalytic intron-exon sequence at 5' of the cDNA sequence of the sense strand of the target dsRNA comprises, or consists of, a nucleotide sequence with a sequence identity of 100% with SEQ ID NO: 15.

SEQ ID NO: 15 (The exon is shown underlined)
CTTCTGTTGATATGGATGCAGTTCACAGACTAAATGTCGGTCGGGGAAGATGTATTCTTCTCATAAGATA
TAGTCGGACCTCTCCTTAATGGGAGCTAGCGGATGAAGTGATGCAACACTGGAGCCGCTGGGAACTAATT
TGTATGCGAAAGTATATTGATTAGTTTTGGAGTACTCGTAAGGTAGCC

In another particular embodiment, the permuted type I autocatalytic intron-exon sequence at 3' of the cDNA sequence of the antisense strand of the target dsRNA comprises, or consists of, a nucleotide sequence with a sequence identity of at least 70, 75, 80, 85, 90, 95, 96, 97, 98, 99% with SEQ ID NO: 16. In a still more particular embodiment, the permuted type I autocatalytic intron-exon sequence at 3' of the cDNA sequence of the sense strand of the target dsRNA comprises, or consists of, a nucleotide sequence with a sequence identity of 100% with SEQ ID NO: 16.

SEQ ID NO: 16 (The exon is shown underlined)
ATGACTCTCTAAATAGCAATATTTACCTTTGGAGGGAAAAGTTATCAGGCATGCACCTGGTAGCTAGTCT
TTAAACCAATAGATTGCATCGGTTTAAAAGGCAAGACCGTCAAATTGCGGGAAAGGGGTCAACAGCCGTT
CAGTACCAAGTCTCAGGGGAAACTTTGAGATGGCCTTGCAAAGGGTATGGTAATAAGCTGACGGACATGG
TCCTAACCACGCAGCCAAGTCCTAAGTCAACAGAT

In a still more particular embodiment, the nucleotide sequence of the invention comprises the sequence SEQ ID NO: 17.

SEQ ID NO: 17
CCCCATAGGGTGGTGTGTGCCACCCCTGATGAGACCGAAAGGTCGAAATGGGGTTTCGCCATGGGTCGGG
ACTTTAAATTCGGAGGATTCGTCCTTTAAACGTTCCTCCAAGAGTCCCTTCCCCAAACCCTTACTTTGTA
AGTGTGGTTCGGCGAATGTACCGTTTCGTCCTTTCGGACTCATCAGGGAAAGTACACACTTTCCGACGGT
GGGTTCGTCGACACCTCTCCCCCTCCCAGGTACTATCCCCTTCTTCTGTTGATATGGATGCAGTTCACAG
ACTAAATGTCGGTCGGGGAAGATGTATTCTTCTCATAAGATATAGTCGGACCTCTCCTTAATGGGAGCTA
GCGGATGAAGTGATGCAACACTGGAGCCGCTGGGAACTAATTTGTATGCGAAAGTATATTGATTAGTTTT
GGAGTACTCGTAAGGTAGCCGCTGACCGAAGCACCGTTGAACCCGAAGGCTAACCGCGAGAAGATGACCC
AGATCATGTTCGAGACCTTCAACTCGCCGGCCATGTATGTGGCCATCCAGATGACTCTCTAAATAGCAAT
ATTTACCTTTGGAGGGAAAAGTTATCAGGCATGCACCTGGTAGCTAGTCTTTAAACCAATAGATTGCATC
GGTTTAAAAGGCAAGACCGTCAAATTGCGGGAAAGGGGTCAACAGCCGTTCAGTACCAAGTCTCAGGGGA
AACTTTGAGATGGCCTTGCAAAGGGTATGGTAATAAGCTGACGGACATGGTCCTAACCACGCAGCCAAGT
CCTAAGTCAACAGATCTTCTGTTGATATGGATGCAGTTCACAGACTAAATGTCGGTCGGGGAAGATGTAT
TCTTCTCATAAGATATAGTCGGACCTCTCCTTAATGGGAGCTAGCGGATGAAGTGATGCAACACTGGAGC
CGCTGGGAACTAATTTGTATGCGAAAGTATATTGATTAGTTTTGGAGTACTCGTAAGGTAGCCCTGGATG
GCCACATACATGGCCGGCGAGTTGAAGGTCTCGAACATGATCTGGGTCATCTTCTCGCGGTTAGCCTTCG
GGTTCAACGGTGCTTCGGTCAGCATGACTCTCTAAATAGCAATATTTACCTTTGGAGGGAAAAGTTATCA
GGCATGCACCTGGTAGCTAGTCTTTAAACCAATAGATTGCATCGGTTTAAAAGGCAAGACCGTCAAATTG
CGGGAAAGGGGTCAACAGCCGTTCAGTACCAAGTCTCAGGGGAAACTTTGAGATGGCCTTGCAAAGGGTA
TGGTAATAAGCTGACGGACATGGTCCTAACCACGCAGCCAAGTCCTAAGTCAACAGATTCAAGGATGTGT

TCCCTAGGAGGGTGGGTGTACCTCTTTTGGATTGCTCCGGCCTTCCAGGAGAGATAGAGGACGACCTCTC
CCCATAGGGTGGTGTGTGCCACCCCTGATGAGACCGAAAGGTCGAAATGGGG

Underlined: the hammerhead ribozyme sequences showing in **bold** the cut site of the ribozyme.

Broken underlined: cDNA from ELVd (C327 to G46 from GeneBank accession number AJ536613.1), showing in double underlined the insertion site of the inverted repeat sequence comprising the sense and antisense strands of the target RNA separated by an intervening sequence comprising an intron type I flanked by exon fragments.

*Italic*: 100 bp of the β-actin gene of *Ceratitis capitata,* in sense and antisense orientation.

Undulating underlined: 5' and 3' exons of the autocatalytic intron of the rRNA 26S gene from *T. thermophila.*

Underlined with dots: Autocatalytic type I intron of the rRNA 26S gene from T. *thermophila.*

Bold: Permuted type I autocatalytic intron-exon sequence at 5' and 3' of the cDNA sequence of the sense and antisense strands, respectively, of the target dsRNA, wherein the exons are showing underlined.

In summary, the nucleotide sequence of the invention allows the production of large amounts of dsRNA. To do this, the cDNAs of the two strands of the target dsRNA are inserted into the cDNA of a plant Avsunviroidae and separated by the cDNA of an autocatalytic type I intron. The co-expression of the resulting RNA together with the tRNA ligase produces a large accumulation of a hybrid molecule in which the dsRNA is fused to the viroid. The autocatalytic intron disappears during the processing of the precursor. In addition, if a permuted type I autocatalytic intron is added to this construction, the dsRNA of interest can be separated in a circular form from the viroid molecule. Figures 1 and 2 illustrate the process disclosed in this paragraph.

As the skilled person in the art understands, the nucleotide sequence of the invention may further comprise regulatory sequences, such as enhances and promoters, origins of replication, ribosomal terminators, resistance genes, etc., which allow the expression of the nucleotide sequence in a host cell.

In another aspect, the present invention relates to a vector, hereinafter "vector of the invention", comprising the nucleotide sequence of the invention.

The term "vector" in accordance with the invention means a plasmid, cosmid, virus, bacteriophage or another vector used conventionally in genetic engineering which carries the nucleotide sequence of the invention. Accordingly, the nucleotide sequence of the invention may be inserted into several commercially available vectors. Nonlimiting examples include prokaryotic plasmid vectors, such as of the pUC-series, pBluescript (Stratagene), the pET-series of expression vectors (Novagen) or pCRTOPO (Invitrogen) and vectors compatible with an expression in mammalian cells like pREP (Invitrogen), pcDNA3 (Invitrogen), pCEP4 (Invitrogen), pMC1neo (Stratagene), pXT1 (Stratagene), pSG5 (Stratagene), EBO-pSV2neo, pBPV-1, pdBPVMMTneo, pRSVgpt, pRSVneo, pSV2-dhfr, plZD35, pLXIN, pSIR (Clontech), pIRES-EGFP (Clontech), pEAK-10 (Edge Biosystems) pTriEx-Hygro (Novagen) and pCINeo (Promega). Samples for plasmid vectors suitable for *Pichia pastoris* include, without being limited to, the plasmids pAO815, pPIC9K and pPIC3.5K (all Invitrogen).

The nucleotide sequence of the invention may be inserted into vectors such that a (further) translational fusion with another nucleotide sequence is generated. The other nucleotide sequence may encode a protein which may, for example, increase the solubility and/or facilitate the purification of the dsRNA encoded by nucleotide sequence of the invention. To generate said fusion of nucleotides, overlap extension PCR can be applied. The products arising therefrom are termed fusion proteins and will be described further below.

The vector of the invention, further to the nucleotide sequence of the invention, may comprise a nucleotide sequence encoding the tRNA ligase. Alternatively, the present invention also encompasses a pair of vectors, the first vector comprising the nucleotide sequence of the invention, and the second vector comprising the nucleotide sequence encoding the tRNA ligase as described in previous paragraphs.

Plasmids which can be used to introduce the nucleotide sequence of the invention into a host cell are pUC18/19 (Roche Biochemicals), pBluescript II, pKK-177-3H (Roche Biochemicals), pBTac2 (Roche Biochemicals), pKK223-3 (Amersham Pharmacia Biotech), pKK-233-3 (Stratagene) or pET (Novagen).

Generally, vectors can contain one or more origins of replication (ori) and inheritance systems for cloning or expression, one or more markers for selection in the host, e.g., antibiotic resistance, and one or more expression cassettes. Suitable origins of replication include, without limiting to, the pUC, p15A, ColE1, the viral SV40 or the M13 origins of replication.

The nucleotide sequences inserted in the vector can, for example, be synthesized by standard methods. Ligation of the nucleotide sequence to transcriptional regulatory elements and/or to other nucleotides sequences can be carried out using established methods well-known by the skilled person in the art. Transcriptional regulatory elements (parts of an expression cassette) ensuring expression in prokaryotes or eukaryotic cells are well known to those skilled in the art. These elements comprise regulatory sequences ensuring the initiation of the transcription (e. g., translation initiation codon, transcriptional termination sequences, promoters, enhancers, and/or insulators) and internal ribosomal entry sites (IRES). Additional regulatory elements may include transcriptional, and/or naturally-associated or heterologous promoter regions. The regulatory elements may be heterologous regulatory elements. Preferably, the nucleotide sequence of the invention is operably linked to such expression control sequences allowing expression in prokaryotes or eukaryotic cells. The co-transfection with a selectable marker such as kanamycin, chloramphenicol or ampicillin resistance genes for culturing in *E. coli* or other bacteria allows the identification and isolation of the transfected cells. Using such markers, the cells are grown in selective medium and those with the highest resistance are the transfected cells.

In another aspect, the invention relates to a host cell, hereinafter "cell of the invention", comprising, e.g., as a result of transformation, transduction, microinjection or transfection, the nucleotide sequence or the vector of the invention.

Techniques for the introduction of nucleic acid into cells are well established in the art and any suitable technique may be employed in accordance with the particular circumstances. For eukaryotic cells, suitable techniques may include calcium phosphate transfection, DEAE- Dextran, electroporation, liposome-mediated transfection and transduction using retrovirus or other virus, e.g. adenovirus, AAV, lentivirus or vaccinia. For bacterial cells, suitable techniques may include calcium chloride transformation, electroporation and transfection using bacteriophage. Marker genes such as antibiotic resistance or sensitivity genes may be used in identifying clones containing nucleic acid of interest, as is well-known in the art. The introduced nucleic acid(s) may be on an extra-chromosomal vector within the cell or the nucleic acid may be integrated into the genome of the host cell. Integration may be promoted by inclusion of sequences within the nucleic acid or vector which promote recombination with the genome, in accordance with standard techniques.

A variety of host-expression systems may be used to express the nucleotide sequence or the vector of the invention. The host cell of the invention may be produced by introducing the nucleotide sequence or vector(s) of the invention into the host cell.

In a particular embodiment, the host cell of the invention comprises
(a) the vector of the invention, or
(c) a pair of vectors, the first vector comprising the nucleotide sequence of the invention, and the second vector comprising the nucleotide sequence encoding the tRNA ligase as described in previous paragraphs.

The host from which the host cell is derived may be any eukaryotic or prokaryote cell. A suitable eukaryotic host cell may be a vertebrate cell, an amphibian cell, a fish cell, an insect cell, a fungal/yeast cell, a nematode cell or a plant cell. The insect cell may be a *Spodoptera frugiperda* cell, a Drosophila S2 cell or a Spodoptera Sf9 cell, the fungal/yeast cell may a *Saccharomyces cerevisiae* cell, *Pichia pastoris* cell or an *Aspergillus* cell.

It is preferred that the vertebrate cell is a mammalian cell such as a human cell, CHO, COS, 293 or Bowes melanoma cell. The plant cell is preferably selected independently from a cell of Anacardium, Anona, Arachis, Artocarpus, Asparagus, Atropa, Avena, Brassica, Carica, Citrus, Citrullus, Capsicum, Carthamus, Cocos, Coffea, Cucumis, Cucurbita, Daucus, Elaeis, Fragaria, Glycine, Gossypium, Helianthus, Heterocallis, Hordeum, Hyoseyamus, Lactuca, Linum, Lolium, Lupinus, Lycopersicon, Malus, Manihot, Majorana, Medicago, Nicotiana, Olea, Oryza, Panieum, Pannesetum, Passiflora, Persea, Phaseolus, Pistachia, Pisum, Pyrus, Prunus, Psidium, Raphanus, Ricinus, Secale, Senecio, Sinapis, Solanum, Sorghum, Theobromus, Trigonella, Triticum, Vicia, Vitis, Vigna and Zea. The cell may be a part of a cell line. The cell from plant may, e.g., be derived from root, leave, bark, needle, bole or caulis.

Suitable prokaryotes (bacteria) useful as hosts for the invention are those generally used for cloning and/or expression include, without being limited to, *E. coli* (e.g., E coli strains BL21, HB101, DH5a, XL1 Blue, Y1090, JM101, and HT115), *Salmonella typhimurium, Serratia marcescens, Burkholderia glumae, Pseudomonas putida, Pseudomonas fluorescens, Pseudomonas stutzeri, Streptomyces lividans, Lactococcus lactis, Mycobacterium smegmatis, Streptomycesor, Bacillus subtilis.* Appropriate culture mediums and conditions for the above described host cells are known in the art.

Preferred examples for host cell to be genetically engineered with the nucleotide sequence or the vector(s) of the invention is a cell of yeast, *E. coli* and/or a species of the genus Bacillus (e.g., *B. subtilis*).

In some embodiments, the host cells may express an exogenous tRNA ligase. For example, the host cells may have been previously transformed with a nucleotide sequence encoding the tRNA ligase. A method as described herein may comprise introducing nucleic acid encoding the nucleotide sequence of the invention into host cells that express an exogenous tRNA ligase. The introduction may be followed by expression of the nucleotide sequence(s) to produce the dsRNA molecule and/or tRNA ligase. In some embodiments, host cells (which may include cells actually transformed although more likely the cells will be descendants of the transformed cells) may be cultured *in vitro* under conditions for expression of the nucleic acid, so that the encoded dsRNA molecule and/or tRNA ligase are produced. When an inducible promoter is used, expression may require the activation of the inducible promoter. The dsRNA molecules may then be recovered from the host cells or the surrounding medium.

In another aspect, the present invention relates to a plant, hereinafter "plant of the invention", comprising the isolated nucleotide sequence of the invention, or the vector of the invention, or the host cell of the invention.

Another aspect of the invention provides a recombinant host cell that expresses the dsRNA molecule and tRNA ligase, as described herein. The host cell may comprise a heterologous nucleotide sequence encoding the tRNA ligase and a heterologous nucleotide sequence encoding the dsRNA molecule (the nucleotide sequence of the invention). The nucleotide sequences may be contained in the same or separate expression vectors as described above. Another aspect of the invention provides a recombinant host cell that expresses an exogenous tRNA ligase and is suitable for transformation with a nucleotide sequence of the invention as disclosed herein. Host cells, chimeric RNA molecules and tRNA ligases are described in more detail above.

The expressed dsRNA, specific of a target RNA, may be isolated and/or purified after production from the host cell and/or culture medium. This may be achieved using any convenient method known in the art. Techniques for the purification and/or the isolation of RNA are well known in the art and include, for example HPLC, FPLC or affinity chromatography.

Following isolation and/or purification, the dsRNA molecule may subsequently be used as desired, e.g. in the formulation of a composition which may include one or more additional components, such as a pharmaceutical or a phytosanitary composition which includes one or more acceptable excipients, vehicles or carriers. dsRNAs produced as described herein may be investigated further, for example the pharmacological properties and/or activity may be determined. Methods and means of RNA analysis are well-known in the art.

In some embodiments, the dsRNA may be modified or adapted after isolated from the host cell. For example, the 2' -OH group of one or more nucleotides in the dsRNA may be chemically modified, for example by addition of a substituent group, such as methyl (e.g. 2 '-O-methyl), halogen (e.g. 2'-Fluoro) or amine (e.g. 2'-NH3), or reduction to 2'-H (e.g. 2' deoxy).

As explained throughout the present description, the nucleotide sequence of the invention allows the production of high amounts of a target dsRNA when it is expressed in a host cell. Thus, in another aspect, the present invention relates to the use of the isolated nucleotide sequence of the invention, or of the vector of the invention, or of the host cell of the invention, or the plant of the invention, for producing a dsRNA specific for a target gene. The terms used for defining this aspect, as well as their particular embodiments, have been explained in previous paragraphs and can be applied to the present inventive aspect. The isolated nucleotide sequence of the invention, or of the vector of the invention, or of the host cell of the invention, or the plant of the invention, may comprise a nucleotide sequence encoding a tRNA ligase. However, as explained in previous paragraphs, the tRNA ligase (or the nucleotide sequence encoding the tRNA ligase) may be present in another vector or in the own genome of the host cell. Thus, in a particular embodiment, the invention relates to the use of the isolated nucleotide sequence of the invention, or of the vector of the invention, or of the host cell of the invention, or the plant of the invention, in combination with a tRNA ligase (or the nucleotide sequence encoding the tRNA ligase), for producing a dsRNA specific for a target gene.

Analogously, in another aspect, the present invention relates to a method for producing dsRNA specific for a target gene, hereinafter "method of the invention", comprising co-expressing in a host cell
a) the nucleotide sequence of the invention and
b) a nucleotide sequence encoding a tRNA ligase,
wherein the nucleotide sequences of a) and b) are in the same or different nucleic acid molecule.

In a particular embodiment of the method of the invention, the host cell is *E. coli.*

In another particular embodiment, the tRNA ligase is the tRNA ligase from eggplant.

The terms "dsRNA", "host cell" and "tRNA ligase" have been defined and explained in previous aspects of the present invention. The co-expression of elements (a) and (b) cited above is carried out by culturing the host cell comprising both elements in suitable conditions. Suitable conditions for culturing a prokaryotic or eukaryotic host cell are well known to the person skilled in the art. Suitable conditions for culturing *E. coli,* are provided in the examples of the present invention. In general, suitable conditions for culturing bacteria are growing them under aeration in Luria Bertani (LB) medium. To increase the yield and the solubility of the expression product, the medium can be buffered or supplemented with suitable additives known to enhance or facilitate both. *E*. *coli* can be cultured from 4 to about 37 °C, the exact temperature or sequence of temperatures depends on the molecule to be overexpressed. In general, *Aspergillus* sp. may be grown on Sabouraud dextrose agar, or potato dextrose agar at about to 10°C to about 40°C, and preferably at about 25°C. Suitable conditions for yeast cultures are known. The skilled person is also aware of all these conditions and may further adapt these conditions to the needs of a particular host species and the requirements of the polypeptide expressed. In case an inducible promoter controls the nucleotide sequence of the invention in the vector present in the host cell, expression of the dsRNA can be induced by addition of an appropriate inducing agent. Suitable expression protocols and strategies are known to the skilled person. Depending on the cell type and its specific requirements, mammalian cell culture can e.g. be carried out in RPMI or DMEM medium containing 10% (v/v) FCS, 2mM L-glutamine and 100 U/ml penicillin/streptomycin. The cells can be kept at 37 °C in a 5% CO2, water saturated atmosphere. Suitable expression protocols for eukaryotic cells are well known to the skilled person.

After culturing the host cell, the dsRNA produced may be isolated and/or purified. Methods of isolation are well-known in the art and comprise, without limitation, steps such as ion exchange chromatography, gel filtration chromatography (size exclusion chromatography), affinity chromatography, high pressure liquid chromatography (HPLC), reversed phase HPLC, or disc gel electrophoresis.

In another aspect, the present invention relates to a phytosanitary composition comprising a dsRNA obtained by the method of the invention.

### BRIEF DESCRIPTION OF THE DRAWINGS

Figure 1 is a representation of the production of dsRNA in *E. coli* with the system derived from ELVd, using the autocatalytic intron of *T. thermophila* to separate the inverted repeats.
Figure 2 is a scheme of the intron-exon permutation strategy (IEP) and of the self-cleavage reaction of the introns of group I, in which the ligation of the exons and the circularization of the dsRNA is produced.
Figure 3 is a representation of a circular double-stranded miniRNA with an extensive perfectly double-stranded region homologous to an endogenous gene of the pest or pathogen of interest, closed in both sides by the fragments of the exons.
Figure 4 shows the sequence and predicted structure of minimum free energy of Eggplant latent viroid (ELVd; GenBank accession number AJ536613.1 (SEQ ID NO: 7)). The hammerhead ribozyme domain and self-cleavage site are indicated on grey background and by an arrowhead, respectively. The position U245-U246 where recombinant RNAs were inserted is indicated by an arrow.

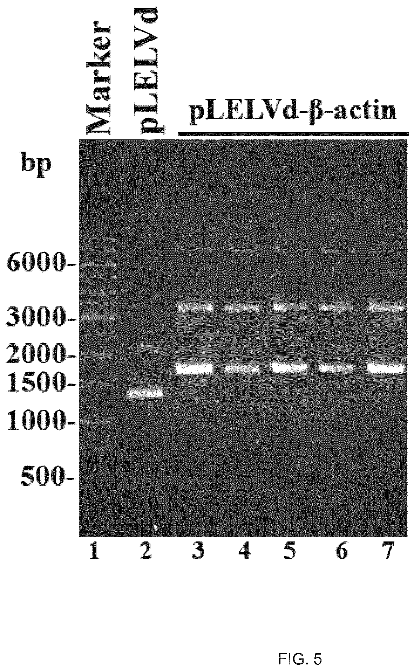

Figure 5 is a 1% agarose gel stained with ethidium bromide in which the electrophoretic migration of the empty plasmid pLELVd (lane 2) is compared to those of five independent clones of pLELVd-β-actin (lanes 3 to 7). Lane 1 corresponds to a ladder of DNA markers whose sizes in base pairs (bp) are indicated on the left.

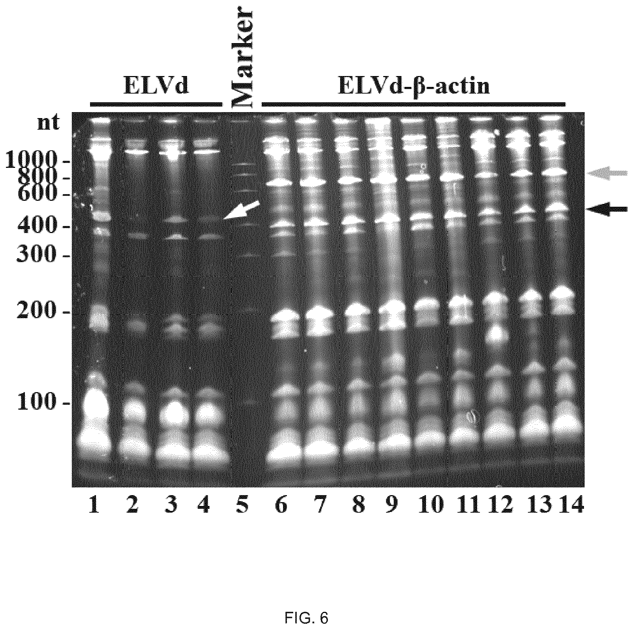

Figure 6 is a 5% polyacrylamide gel, containing 8 M urea, stained with ethidium bromide in which total Escherichia coli RNA preparations were analyzed. Lanes 1 to 4 correspond to E. coli clones transformed with empty pLELVd, lane 5 corresponds to a ladder of RNA markers whose size in nucleotides (nt) is indicated on the left, lanes 6 to 7 correspond to E. coli clones transformed with pLELVd-β-actin. White, gray and black arrows point to ELVd RNA, ELVd-dsRNA-β-actin, and the spliced intron RNA, respectively.

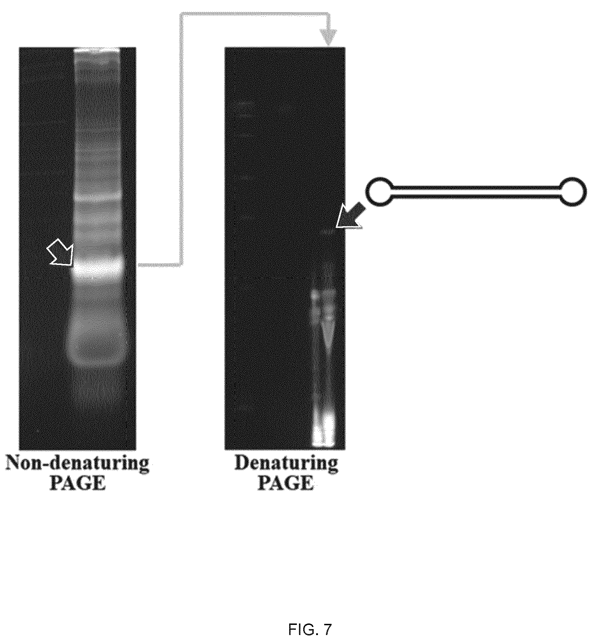

Figure 7 are two 5% polyacrylamide gels, the second containing 8 M urea, stained with ethidium bromide. A total RNA preparation from an Escherichia coli clone transformed with pLELVd-β-actin was separated in the first gel (left) under non-denaturing conditions. After staining the gel, the band pointed by the arrow was cut from the gel and applied onto a second gel (right) that was run under denaturing conditions. The arrow on the right points to the spliced circular dsRNA, which is also schematized in the figure. Both gels contain RNA markers on the left.

### EXAMPLES

Due to the instability of DNA inverted repeats in *E. coli* cells when plasmids to produce dsRNA to induce RNAi in pests and pathogens were built, bacterial colonies bearing the expected constructs were not obtained. In order to alleviate the well-known problems with DNA inverted repeats in *E. coli,* the cDNAs corresponding to the two strands of the target dsRNAs were separated with a 433-bp cDNA (SEQ ID NO: 6) corresponding to *T. thermophila* 26S rRNA Group-I intron (GenBank accession number V01416.1 [SEQ ID NO: 23]) plus 10 bp of both native flanking exons. In this way, while the intron cDNA will separate the inverted repeat at the DNA level, the intron would self-splice from the transcript allowing formation of the dsRNA.

For this purpose, we amplified by PCR using the Phusion high-fidelity DNA polymerase (Thermo Scientific) two different DNAs corresponding to a 100-bp fragment of C. *capitata* beta-actin mRNA (GenBank accession number EU665679.1 [SEQ ID NO: 24]) in both orientations (SEQ ID NO: 1 and SEQ ID NO: 2). We also amplified by PCR a DNA corresponding to *T. thermophila* 26S rRNA group-I intron with fragments of flanking exons (SEQ ID NO: 6), from a plasmid obtained by gene synthesis. PCR products were separated by electrophoresis in a 1% agarose gel and the gel stained with ethidium bromide. DNAs of the expected size were eluted from the gel using silicagel spin columns. The three DNAs were assembled between the positions T245 and T246 of the ELVd-AJ536613.1 cDNA with duplicated hammerhead ribozyme domains (SEQ ID NO: 11) in plasmid pLELVd (Daròs, J.A., Aragonés, V., and Cordero, T. (2018) Sci. Rep., 8,1904), by means of the Gibson assembly reaction using the NEBuilder HiFi DNA assembly master mix (New England Biolabs). Competent *E. coli* cells were finally electroporated with the products of the Gibson assembly reactions and transformed bacteria selected in Luria-Bertani (LB) plates containing 50 µg/ml ampicillin. We grew five independent *E. coli* clones in liquid LB cultures and purified the corresponding plasmids. An analysis by electrophoresis in a 1% agarose gel that was stained with ethidium bromide showed all of them being the same size and exhibiting a migration delay consistent with the inserted DNAs (Figure 5, compare lane 2 with lanes 3 to 7). The expected sequence SEQ ID NO: 12) was experimentally confirmed in one of these plasmids. This plasmid was used to co-electroporate the RNase III-deficient HT115(DE3) strain of *E. coli* that lacks RNase III (Timmons, L., Court, D.L., and Fire, A. (2001) Gene, 263, 103-112), along with plasmid pLtRnlSm (Daròs *et al.,* 2018, cited *ad supra)* from which eggplant tRNA ligase (SEQ ID NO: 13 and SEQ ID NO: 14) is constitutively expressed. As controls, pLtRnlSm was also co-electroporated with the plasmid to express the empty ELVd (pLELVd) (Daròs *et al.,* 2018, cited *ad supra).* Nine independent colonies (four for the empty ELVd control) were picked from LB plates containing 50 µg/ml ampicillin and 34 µg/ml chloramphenicol and grown for 24 hours in liquid Terrific Broth (TB) medium. Total RNA was extracted from the cells using a 1:1 mix of phenol and chloroform (pH 8.0) (Daròs *et al.,* 2018), and analyzed by polyacrylamide gel electrophoresis (PAGE) in denaturing conditions (8 M urea) (Daròs *et al.,* 2018, cited *ad supra).* Two prominent bands above the 600-nt and slightly below the 400-nt RNA markers were observed in the RNA extracts from bacteria transformed with the plasmid to express the dsRNA (Figure 6, lanes 6 to 14, gray and black arrows respectively). Remarkably, these bands exhibited a fluorescence intensity comparable to those from the endogenous *E. coli* rRNAs (Figure 6, upper part of the gel), indicating a large accumulation in *E. coli.* RNA extracts from the empty ELVd controls exhibited a single prominent band above the 400-nt marker that, according to our previous analysis (Fadda, Z., Daròs, J.A., Fagoaga, C., Flores, R., and Duran-Vila, N. (2003) J. Virol., 77), corresponds to the 333-nt-long circular ELVd RNA (Figure 6, lanes 1 to 4, white arrow). Circular RNAs migrate slower than the linear counterparts of the same size in denaturing conditions. The identity of the different RNA species indicated in the Figure 2 was confirmed by Northern-blot hybridization analysis using ³²P-labelled RNA probes complementary to the ELVd and the *T. thermophila* intron RNAs.

Taken together, these results indicate that, while the *T. thermophila* cDNA serves to stabilize the inverted repeats in the *E. coli* expression plasmids, the intron very efficiently self-splices from the primary transcript in bacterial cells facilitating the accumulation of large amounts of a circular RNA product that consists of an ELVd scaffold from which the dsRNA bulges. This is schematized in Figure 1. The dsRNAs produced in *E. coli* with this method showed the same insecticide activities as the counterparts of the same sequence obtained using the standard methodologies, such as two-strand transcription from two opposite promoters.

Next, we designed a novel strategy to separate the active dsRNA from the undesired ELVd scaffold. For this purpose, we took advantage of the permuted intron-exon (PIE) reaction (Price, J. V, Engberg, J., and Cech, T.R. (1987) J. Mol. Biol., 196, 49-60; Puttaraju, M. and Been, M. (1992) Nucleic Acids Res., 20, 5357-5364). We built a new plasmid in which a cDNA corresponding to the *T. thermophila* 26S rRNA group-I intron with 10 nt of the flanking exons (SEQ ID NO: 6) was split and permuted, and the 3' fragment (SEQ ID NO: 15) inserted between the 5' moiety of ELVd and the plus strand of the dsRNA, and the 5' fragment (SEQ ID NO: 16) inserted between the minus strand of the dsRNA and the 3' moiety of the ELVd (see Figure 2). We built this plasmid by amplifying the DNAs by PCR with the Phusion high-fidelity DNA polymerase and assembling the fragment by the Gibson reaction. The sequence of the plasmid was confirmed by sequencing. The selected plasmid, along pLtRnlSm to co-express the eggplant tRNA ligase, was used to electroporate competent *E. coli* cells. Transformed bacteria were selected in LB plates containing ampicillin and chloramphenicol. Next, we grew liquid cultures in TB, containing the same antibiotics, and extracted total RNA from bacteria using phenol:chloroform. The RNA preparation was analyzed by PAGE under non-denaturing and denaturing (8 M urea) conditions (Figure 7). The alteration of electrophoretic migration from non-denaturing to denaturing conditions confirmed the production in *E. coli* of a circular RNA molecule consisting of a 100-bp dsRNA locked at both ends by the exon fragments (Figure 3), as a result of the self-cleavage of the regular intron I and the permuted intron I (Figure 2).

In conclusion, we have worked out a new method to produce dsRNA that can be used in RNAi-based biotechnology applications. The new method is based on the activity of two versions of a self-splicing group-I intron, the second in a permuted form. The first intron stabilizes the DNA inverted repeats that are required to transcribe a dsRNA in *E. coli,* while is efficiently processed from the final transcript. The second (permuted) intron cleaves the dsRNA from the viroid scaffold producing a circular molecule consisting of the dsRNA locked at both ends by the exon fragments (Figure 3).

## Claims

1. An isolated nucleotide sequence comprising
a) the cDNAs of two strands of a target dsRNA separated by a type I autocatalytic intron flanked by exons or exon fragments, and
b) a plant Avsunviroidae sequence,
- wherein element (a) is inserted in the plant Avsunviroidae sequence.

2. Isolated nucleotide sequence according to claim 1, further comprising type I permuted autocatalytic intron-exon sequences between the plant Avsunviroidae sequence and the cDNAs sequences.

3. Isolated nucleotide sequence according to claim 1 or 2, wherein the type I autocatalytic intron is the type I autocatalytic intron of the rRNA 26S from *Tetrahymena thermophila.*

4. Isolated nucleotide sequence according to anyone of claims 1 to 3, wherein the Avsunviroidae sequence is the *Eggplant latent viroid* (ELVd) sequence, the Avocado sunblotch viroid (ASBVd) sequence, the Peach latent mosaic viroid (PLMVd) sequence, or the Chrysanthemum chlorotic mottle viroid (CChMVd) sequence.

5. Isolated nucleotide sequence according to anyone of claims 1 to 4, wherein the target RNA is a gene from a plant pathogen or pest, or the β-actin gene from *Ceratitis capitata.*

6. Isolated nucleotide sequence according to anyone of claims 1 to 5, further comprising a nucleotide sequence encoding a tRNA ligase.

7. Isolated nucleotide sequence according to claim 6, wherein the tRNA ligase is the tRNA ligase from eggplant.

8. A vector comprising an isolated nucleotide sequence according to anyone of claims 1 to 7.

9. A host cell comprising an isolated nucleotide sequence according to anyone of claims 1 to 7 or a vector according to claim 8.

10. Use of an isolated nucleotide sequence according to anyone of claims 1 to 7, or a vector according to claim 8, or a host cell according to claim 9, for producing dsRNA specific for a target gene.

11. A method for producing dsRNA specific for a target gene comprising co-expressing in a host cell
a) a nucleotide sequence according to anyone of claims 1 to 5 and
b) a nucleotide sequence encoding a tRNA ligase,
wherein the nucleotide sequences of a) and b) are in the same or different nucleic acid molecule.

12. A method according to claim 11, wherein the host cell is *E. coli.*

13. A method according to claim 11 or 12, wherein the tRNA ligase is the tRNA ligase from eggplant.

## Patentansprüche

1. Isolierte Nukleotidsequenz, umfassend
a) die cDNA zweier Stränge einer Ziel-dsRNA, die durch ein autokatalytisches Intron vom Typ I, das von Exons oder Exonfragmenten flankiert ist, getrennt sind, und
b) eine Pflanzen-Avsunviroidae-Sequenz,
- wobei Element (a) in die Pflanzen-Avsunviroidae-Sequenz eingefügt wird.

2. Isolierte Nukleotidsequenz nach Anspruch 1, ferner umfassend permutierte autokatalytische Intron-Exon-Sequenzen vom Typ I zwischen der Pflanzen-Avsunviroidae-Sequenz und den cDNA-Sequenzen.

3. Isolierte Nukleotidsequenz nach Anspruch 1 oder 2, wobei das autokatalytische Intron vom Typ I das autokatalytische Intron vom Typ I der rRNA 26S aus *Tetrahymena thermophila* ist.

4. Isolierte Nukleotidsequenz nach einem der Ansprüche 1 bis 3, wobei die Avsunviroidae-Sequenz die ELVd-Sequenz (*Eggplant latent viroid,* ELVd), die ASBVd-Sequenz (Avocado sunblotch viroid, ASBVd), die PLMVd-Sequenz (Peach latent mosaic viroid, PLMVd) oder die CChMVd-Sequenz (Chrysanthemum chlorotic mottle viroid, CChMVd) ist.

5. Isolierte Nukleotidsequenz nach einem der Ansprüche 1 bis 4, wobei die Ziel-RNA ein Gen aus einem Pflanzenpathogen oder -schädling oder das β-Actin-Gen aus *Ceratitis capitata* ist.

6. Isolierte Nukleotidsequenz nach einem der Ansprüche 1 bis 5, ferner umfassend eine Nukleotidsequenz, die eine tRNA-Ligase codiert.

7. Isolierte Nukleotidsequenz nach Anspruch 6, wobei die tRNA-Ligase die tRNA-Ligase aus Auberginen ist.

8. Vektor, umfassend eine isolierte Nukleotidsequenz nach einem der Ansprüche 1 bis 7.

9. Wirtszelle, umfassend eine isolierte Nukleotidsequenz nach einem der Ansprüche 1 bis 7 oder Vektor nach Anspruch 8.

10. Verwendung einer isolierten Nukleotidsequenz nach einem der Ansprüche 1 bis 7 oder eines Vektors nach Anspruch 8 oder einer Wirtszelle nach Anspruch 9 zur Herstellung einer für ein Zielgen spezifischen dsRNA.

11. Verfahren zur Herstellung von für ein Zielgen spezifischer dsRNA, umfassend die Koexpression von Folgendem in einer Wirtszelle:
a) einer Nukleotidsequenz nach einem der Ansprüche 1 bis 5 und
b) einer Nukleotidsequenz, die eine tRNA-Ligase codiert,
wobei die Nukleotidsequenzen von a) und b) in demselben oder einem anderen Nukleinsäuremolekül vorliegen.

12. Verfahren nach Anspruch 11, wobei die Wirtszelle *E*. *coli* ist.

13. Verfahren nach Anspruch 11 oder 12, wobei die tRNA-Ligase die tRNA-Ligase aus Auberginen ist.

## Revendications

1. Séquence nucléotidique isolée comprenant
a) les ADNc de deux brins d'un ARNdb cible séparés par un intron autocatalytique de type I flanqué d'exons ou de fragments d'exons, et
b) une séquence de viroïde végétal de la famille des *Avsunviroidae,*
- dans laquelle l'élément (a) est inséré dans la séquence de viroïde végétal de la famille des *Avsunviroidae.*

2. Séquence nucléotidique isolée selon la revendication 1, comprenant en outre des séquences intron-exon autocatalytiques permutées de type I entre la séquence de viroïde végétal de la famille des *Avsunviroidae* et les séquences d'ADNc.

3. Séquence nucléotidique isolée selon la revendication 1 ou 2, dans laquelle l'intron autocatalytique de type I est l'intron autocatalytique de type I de l'ARNr 26S de *Tetrahymena thermophila.*

4. Séquence nucléotidique isolée selon l'une des revendications 1 à 3, dans laquelle la séquence *Avsunviroidae* est la séquence du viroïde latent de l'aubergine (ELVd), la séquence du viroïde de la tache solaire de l'avocat (ASBVd), la séquence du viroïde latent de la mosaïque du pêcher (PLMVd), ou du viroïde du chrysanthème à marbrure chlorotique (CChMVd).

5. Séquence nucléotidique isolée selon l'une des revendications 1 à 4, gène d'un agent pathogène ou d'un ravageur de plante, ou le gène de la β-actine de *Ceratitis capitata.*

6. Séquence nucléotidique isolée selon l'une des revendications 1 à 5, comprenant en outre une séquence de nucléotides codant pour une ligase d'ARNt.

7. Séquence nucléotidique isolée selon la revendication 6, dans laquelle l'ARNt ligase est l'ARNt ligase de l'aubergine.

8. Vecteur comprenant une séquence nucléotidique isolée selon l'une quelconque des revendications 1 à 7.

9. Cellule hôte comprenant une séquence nucléotidique isolée selon l'une quelconque des revendications 1 à 7 ou un vecteur selon la revendication 8.

10. Utilisation d'une séquence nucléotidique isolée selon l'une quelconque des revendications 1 à 7, ou d'un vecteur selon la revendication 8, ou d'une cellule hôte selon la revendication 9, pour la production d'ARNdb spécifique d'un gène cible.

11. Procédé de production d'ARNdb spécifique d'un gène cible comprenant la co-expression dans une cellule hôte
a) une séquence de nucléotides selon l'une quelconque des revendications 1 à 5 et
b) une séquence de nucléotides codant pour une ARNt ligase,
dans lequel les séquences nucléotidiques de a) et b) sont dans la même molécule d'acide nucléique ou différente.

12. Procédé selon la revendication 11, dans lequel la cellule hôte est *E*. *coli.*

13. Procédé selon la revendication 11 ou 12, dans laquelle l'ARNt ligase est l'ARNt ligase de l'aubergine.
